(19) Europäisches Patentamt
European Patent Office
Office européen des brevets

(11) Veröffentlichungsnummer: **0 072 489**
**B1**

(12) # EUROPÄISCHE PATENTSCHRIFT

(45) Veröffentlichungstag der Patentschrift: **07.05.86**

(21) Anmeldenummer: **82107020.8**

(22) Anmeldetag: **04.08.82**

(51) Int. Cl.⁴: **G 03 C 5/54**, G 03 C 7/26, G 03 C 7/00, C 07 C147/06, C 07 C147/14, C 07 C149/30, C 07 C149/32

(54) **Fotografisches Material mit einer durch organische Farbstoffe anfärbbaren Schicht.**

(30) Priorität: **17.08.81 DE 3132333**

(43) Veröffentlichungstag der Anmeldung: **23.02.83 Patentblatt 83/08**

(45) Bekanntmachung des Hinweises auf die Patenterteilung: **07.05.86 Patentblatt 86/19**

(84) Benannte Vertragsstaaten: **BE CH DE FR GB LI**

(56) Entgegenhaltungen:
**DE-A- 2 009 112**
**DE-A- 2 302 230**
**DE-A- 2 321 329**
**DE-A- 2 937 294**
**GB-A- 858 890**

(73) Patentinhaber: **AGFA-GEVAERT Aktiengesellschaft**
**D-5090 Leverkusen 1 (DE)**

(72) Erfinder: **Bergthaller, Peter, Dr.**
**Wolfskaul 1**
**D-5000 Köln 80 (DE)**
Erfinder: **Strauss, Jürgen, Dr.**
**Andreas-Gryphius-Strasse 24**
**D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 072 489 B1

**Beschreibung**

Die Erfindung betrifft ein fotografisches Material mit einer auf einem Schichtträger angeordneten durch organische Farbstoffe anfärbbaren Schicht, die ein Metallisierungsmittel für die Bildung von organischen Farbstoff-Metall-Komplexen enthält. Insbesondere betrifft die Erfindung ein Bildempfangselement für die Herstellung farbfotografischer Bilder nach dem Farbdiffusionsübertragungsverfahren, wobei für die Erzeugung des Farbbildes nachmetallisierbare diffusionsfähige Farbstoffe zur Anwendung gelangen. Als Metallisierungsmittel dienen erfindungsgemäß in der anfärbbaren Schicht enthaltene Nickelkomplexe von 2,2'-Thiobisphenolen, 2,2'-Sulfinylbisphenolen oder 2,2'-Sulfonylbisphenolen.

In Bildempfangsschichten für das fotografische Farbdiffusionsübertragungsverfahren ist die Verwendung von mehrwertigen, insbesondere komplexbildenden Metallkationen von besonderem Interesse. Bekanntlich wird bei dem besonders für die Colorsofortbildfotografie wichtigen Farbdiffusionsübertragungsverfahren ein lichtempfindliches Aufzeichnungsmaterial mit mehreren Silberhalogenidemulsionsschichten unterschiedlicher Spektralempfindlichkeit und diesen zugeordneten farbgebenden Verbindungen verwendet. Als farbgebende Verbindungen kommen beispielsweise sogenannte Farbstoffentwickler (« dye developer ») in Frage, wobei es sich um originär diffusionsfähige Verbindungen mit einem chromophoren Rest handelt und einer Entwicklerfunktion, durch die die Verbindungen bei der Entwicklung bildmäßig immobilisiert werden, oder auch nicht-diffundierende farbgebende Verbindungen mit einem chromophoren Rest, der bei Entwicklung bildmäßig als diffusionsfähiger Farbstoff oder Farbstoffvorläufer in Freiheit gesetzt wird (Farbabspalter). Sofern der chromophore Rest der Farbstoffentwickler oder der Farbabspalter besondere Substituenten enthält, die als Liganden zur Komplexbildung mit mehrwertigen Metallkationen geeignet sind, besteht die Möglichkeit, die Komplexbildung zur Verbesserung der Lichtstabilität, zur Beeinflussung des Farbtones sowie zur Fixierung der in die Bildempfangsschicht übertragenen Bildfarbstoffe auszunutzen. Zweckmäßigerweise wird die Komplexierung erst nach erfolgtem Farbübertrag durchgeführt, wozu entweder das Bildempfangsmaterial mit einer Lösung der komplexbildenden mehrwertigen Metallkationen, z. B. in Form einer Lösung der entsprechenden Salze, behandelt wird oder dadurch, daß die mehrwertigen Metallkationen in eine oder mehrere Schichten des Bildempfangsmaterials eingelagert werden, so daß sie sofort während des Entwicklungsvorganges mit den eindiffundierenden Farbstoffen unter Komplexbildung reagieren können.

In Research Disclosure Nr. 18534 (September 1979) sind Polymere mit einem Gehalt an Komplex gebundenen mehrwertigen Metallkationen und deren Verwendung in Bildempfangsschichten von farbfotografischen Materialien für das Farbdiffusionsübertragungsverfahren beschrieben. Die Aufnahmekapazität der dort erwähnten Polymerisate für mehrwertige Metallkationen ist jedoch begrenzt.

Aus EP-A 0 009 411 ist es bekannt, daß als Metallisierungsmittel für nachkomplexierbare Bildfarbstoffe die kationischen Nickel- oder Kupferkomplexe von Polyvinylpyridin oder Polyvinylimidazol verwendet werden können. Diese Komplexe weisen zwar bei neutralem pH nur wenig Eigenfärbung auf, sie nehmen aber beim pH-Wert eines alkalischen Verarbeitungsmediums rasch eine rötlichgelbe Färbung an, die nur sehr langsam — in der Regel über Tage — wieder abgebaut wird und das Bild während dieser Zeit unansehnlich macht. Zudem beeinflußt der Anteil an koordinierten heteroaromatisch gebundenen N-Atomen, der am Farbstoff-Metallkomplex noch gebunden bleibt, die Nuance des Bildtones, insbesondere bei den blaugrünen Farbstoffkomplexen, in nachteiliger Weise. In den meisten Fällen kommt es zu einer unerwünschten Kurzverschiebung der Absorptionsbande, durch die der Farbton nach blau verschoben wird.

Aus der genannten europäischen Patentanmeldung ist weiterhin bekannt, daß als Metallisierungsmittel Metallkomplexe von Polymeren mit Iminodiessigsäureeinheiten verwendet werden können. Diese Metallisierungsmittel sind polymer-chemisch schwer zu handhaben und — insbesondere im Fall von Latexpolymeren — agglomerationsanfällig.

Der Erfindung liegt die Aufgabe zugrunde, ein Metallisierungsmittel anzugeben, das auf besonders einfache Weise in eine anfärbbare Schicht eines fotografischen Materials, insbesondere eines Bildempfangselementes für farbfotografische Bilder nach dem Diffusionsverfahren eingebracht werden kann, das Metallionen, im speziellen Fall Nickelionen, in diffusionsfester Form gebunden enthält, mit kationischen Beizmitteln nicht agglomeriert und keinerlei Nachteile für das entstehende Bild, insbesondere keine Eigenfärbung, besitzt.

Es wurde gefunden, daß Metallisierungsmittel, die den geschilderten Ansprüchen genügen, auf besonders einfache Weise hergestellt werden können, wenn man 2,2'-Thiobisphenole, 2,2'-Sulfinylbisphenole oder 2,2'-Sulfonylbisphenole, gegebenenfalls in Gegenwart von Hilfsbasen mit Nickelsalzen zur Umsetzkung bringt. Die gebildeten wasserunlöslichen Nickelkomplexverbindungen können in Form wäßriger Dispersionen gegebenenfalls in Gegenwart hochsiedender wasserunlöslicher Ölbildner den Gießlösungen für die anfärbbaren Schichten zugesetzt werden.

Gegenstand der Erfindung ist ein fotografisches Material mit einer auf einem Schichträger angeordneten durch organische Farbstoffe anfärbbaren Schicht, die ein Metallisierungsmittel für die Bildung von organischen Farbstoff-Metall-Komplexen enthält, dadurch gekennzeichnet, daß die durch organische Farbstoffe anfärbbare Schicht als Metallisierungsmittel einen aus einem 2,2'-Bisphenol einer

der folgenden allgemeinen Formeln I und II gebildeten wasserunlöslichen Nickelkomplex enthält :

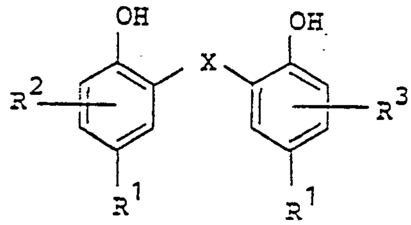

(I)

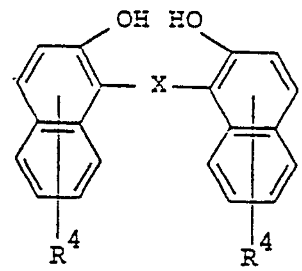

(II)

worin bedeuten

X 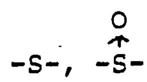 oder —$SO_2$— ;

$R^1$ einen Kohlenwasserstoffrest mit bis zu 18 C-Atomen, der entweder direkt oder über —O— angeknüpft ist, Halogen, insbesondere Chlor oder eine Gruppe $R^4$ ;

$R^2$ Wasserstoff, Halogen, insbesondere Chlor, Alkyl oder Alkoxy mit bis zu 18 C-Atomen, Alkenyl, z. B. Allyl oder Methallyl, einen Rest zur Vervollständigung eines ankondensierten Benzolringes ; oder eine Gruppe $R^4$, falls nicht $R^1$ eine Gruppe $R^4$ bedeutet,

$R^3$ einen Rest wie definiert für $R^2$ oder eine Gruppe der Formel

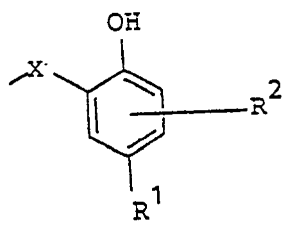

worin X, $R^1$ und $R^2$ die angegebene Bedeutung haben,

$R^4$ $-CO-O-R^5$, 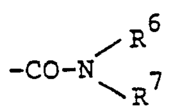 , 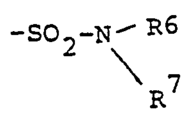 ,

—NH—CO—$R^8$ oder —NH—$SO_2R^9$ ;

$R^5$ Alkyl, Aralkyl, oder Cycloalkyl mit bis zu 8 C-Atomen ;

$R^6$, $R^7$ Wasserstoff, Alkyl, Aralkyl, Aryl, Cycloalkyl oder gemeinsam einen Rest zur Vervollständigung einer 5-, 6- oder 7-gliedrigen cyclischen Aminogruppe (Pyrrolidin, Piperidin, Perhydroazepin, Morpholin) ;

$R^8$ Alkyl, Aralkyl, Aryl, —$OR^5$ oder

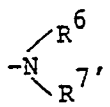,

$R^9$ Alkyl, Aryl oder

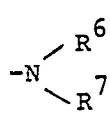.

Die in den Formeln I und II dargestellten Reste $R^1$, $R^2$, $R^3$ und $R^4$ haben insbesondere die Funktion von Ballastresten, d. h. die Gesamtheit der in einer Verbindung vorliegenden Reste $R^1$, $R^2$, $R^3$ (I) bzw. $R^4$ (II) ist hinsichtlich der Größe derart beschaffen, daß eine diffusionsfeste Einlagerung der aus den Verbindungen gebildeten Nickelkomplexe in fotografische Schichten gewährleistet ist.

Als Kohlenwasserstoffreste, die durch $R^1$ dargestellt sind, kommen beispielsweise in Frage : Alkyl, Aralkyl, Cycloalkyl oder Aryl. Besondere Beispiele sind n-Butyl, tert.-Butyl, tert.-Amyl, tert.-Octyl, n-Dodecyl, 1-Phenylethyl, 1-Phenylisopropyl, Cyclohexyl.

Für den Fall, daß $R^2$ einen Rest zur Vervollständigung eines ankondensierten Benzolringes bedeutet, stellt $R^1$ vorzugsweise eine Gruppe $R^4$ dar. Im übrigen ist für die durch $R^2$ dargestellten Reste die Position benachbart zu OH bevorzugt. Falls $R^3$ von $R^2$ verschieden ist, bedeutet $R^2$ bevorzugt Wasserstoff. In Formel II ist $R^4$ bevorzugt eine Sulfamoylgruppe in 6-Stellung des Naphthalinringes.

Typische für die Herstellung von als Metallspender in anfärbbaren Schichten geeigneten Nickelchelaten verwendbare Bisphenole sind nachstehend aufgeführt.

Verbindung 1

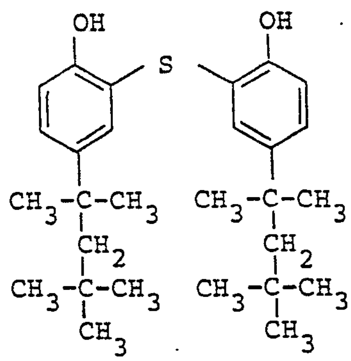

Verbindung 2

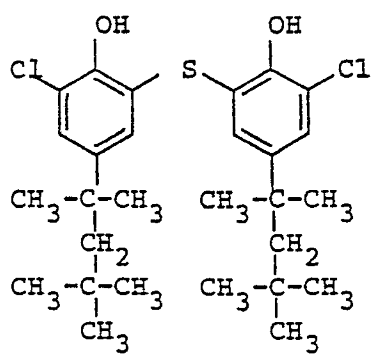

Verbindung 3

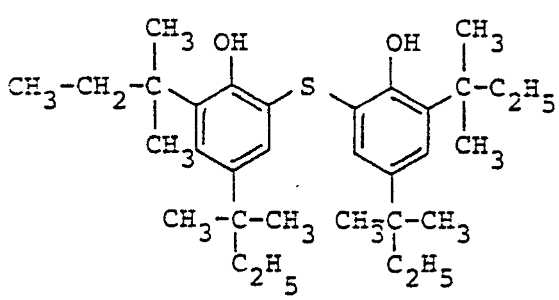

Verbindung 4

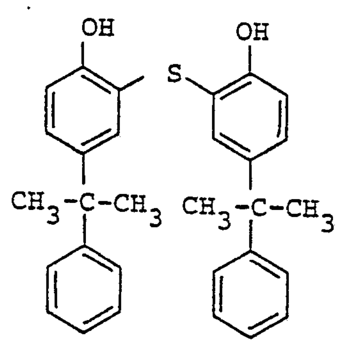

(Fortsetzung)

Verbindung 5

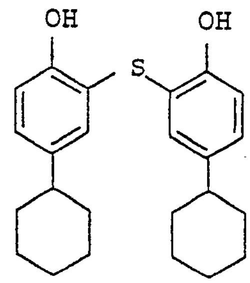

Verbindung 6

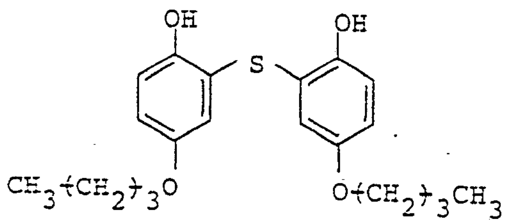

Verbindung 7

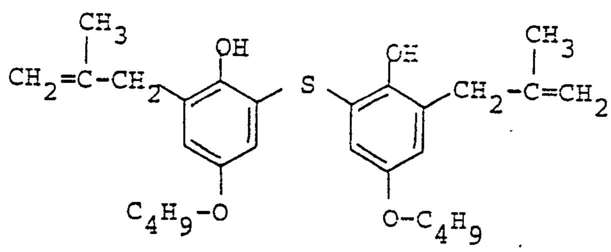

Verbindung 8

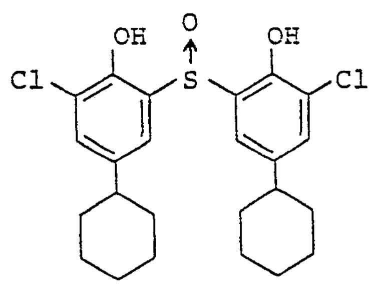

Verbindung 9

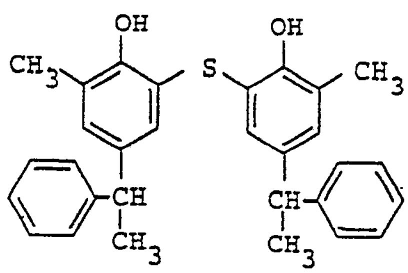

Verbindung 10

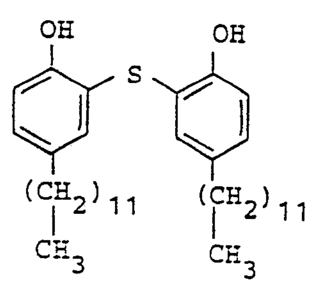

(Fortsetzung)

Verbindung 11

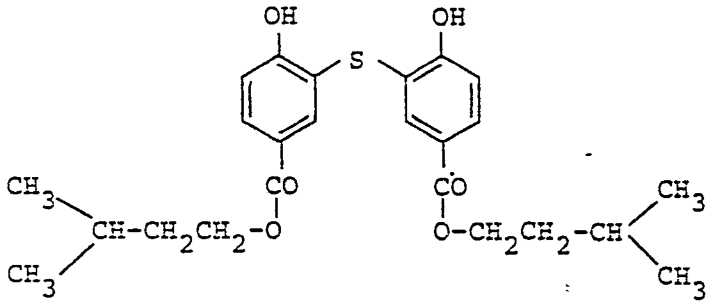

Verbindung 12

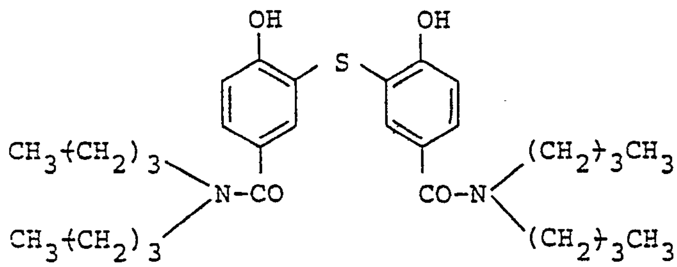

Verbindung 13

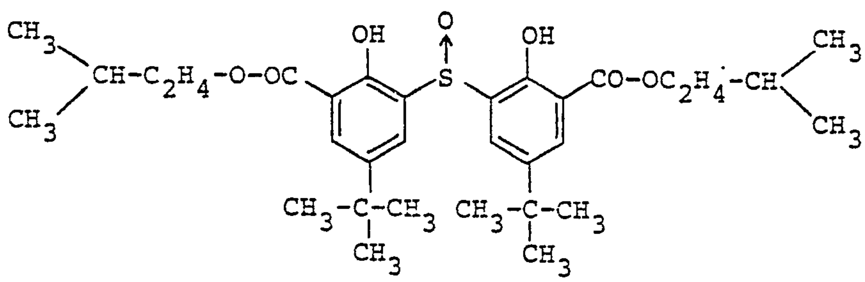

Verbindung 14

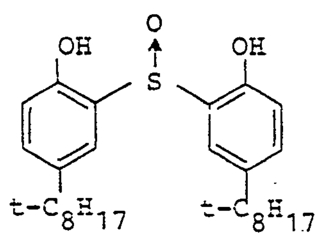

Verbindung 15

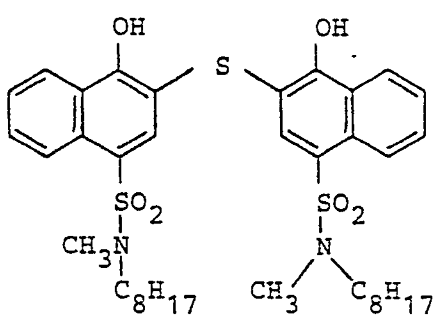

(Fortsetzung)

Verbindung 16

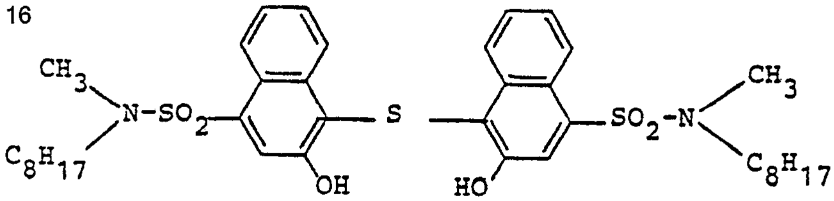

Verbindung 17

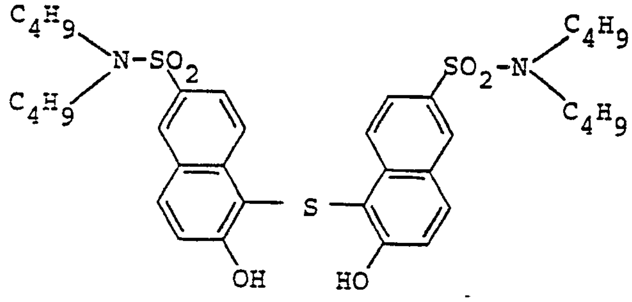

Verbindung 18

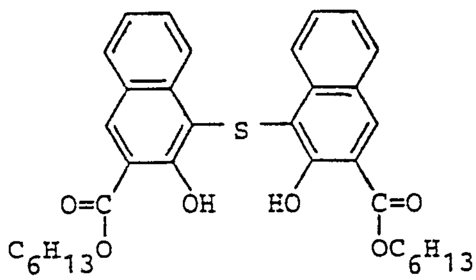

Verbindung 19

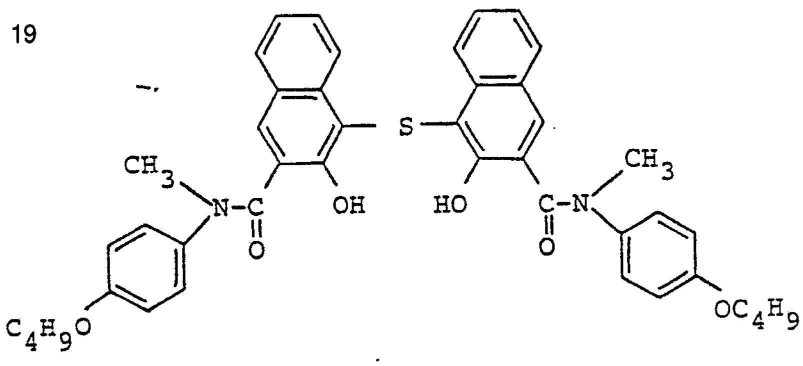

Verbindung 20

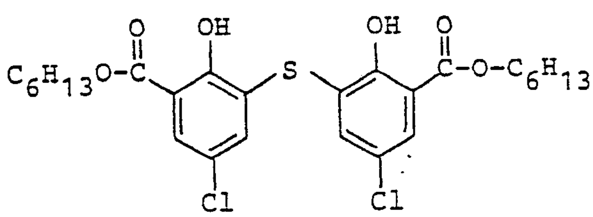

(Fortsetzung)

Verbindung 21

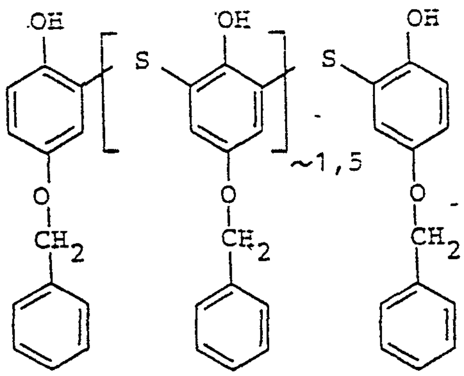

Verbindung 22

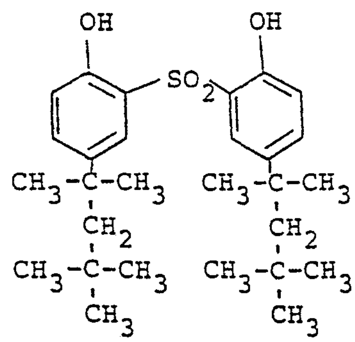

Die Herstellung der erfindungsgemäßen 2,2'-Thiobisphenole ist grundsätzlich bekannt und z. B. in GB 858 890 sowie in DE-OS 29 37 294 beschrieben. Alle aufgeführten 2,2'-Thiobisphenole wurden nach dem dort angegebenen Verfahren durch Umsetzung des entsprechenden Phenols mit der 0,5 molaren Menge Schwefeldichlorid in Gegenwart von wenig $ZnCl_2$ hergestellt. Falls das eingesetzte Phenol zwei unsubstituierte o-Stellungen zur phenolischen Hydroxylgruppe aufweist und Schwefeldichlorid im Überschuß eingesetzt wird, werden Umsetzungsprodukte erhalten, die neben den Thiobisphenolen (Formel I, $R^2 = R^3 = H$) höhermolekulare Verbindungen mit 2, 3 oder mehr Thioetherbrücken und entsprechend 3, 4 oder mehr Phenolresten enthalten. Die Sulfoxide und Sulfone sind aus den Thiobisphenolen durch milde Oxidation mit $H_2O_2$ in Eisessig oder in Dimethylformamid bei Gegenwart von Wolframsäure herzustellen. Sie sind in Methanol besser löslich als die Thiobisphenole.

Herstellung der Verbindung 7

Verbindung 6 wurde in Dioxan nach Zugabe von 2 Äquivalenten Natriumhydrid bei Raumtemperatur mit 2 Äquivalent Methallylchlorid umgesetzt und anschließend 7 h auf 105 °C gehalten. Vom ausgeschiedenen NaCl wurde abgesaugt und das Lösungsmittel wurde bis zu einer Gutstemperatur von 150 °C abdestilliert.

Die in den Metallisierungsmitteln gemäß der Erfindung vorliegenden wasserunlöslichen Nickelkomplexe enthalten pro Mol Bisphenol (I, II) 0,5-1,5 Mol Nickel ; sie entsprechen im Fall der Bisphenole I beispielsweise den allgemeinen Formeln III, IV oder V, worin die angegebenen Reste die oben beschriebene Bedeutung besitzen und L, L' für nicht farbige neutrale (L) oder anionische (L') Fremdliganden stehen, die bei der Herstellung der Komplexe mit eingebracht werden.

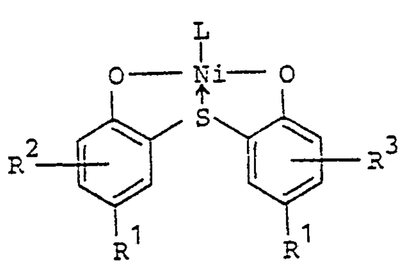 (III)

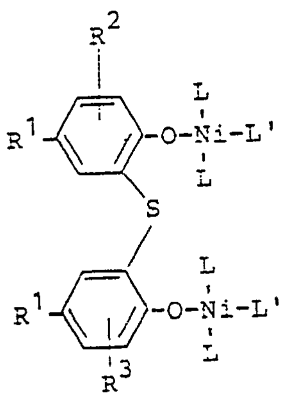

(IV)

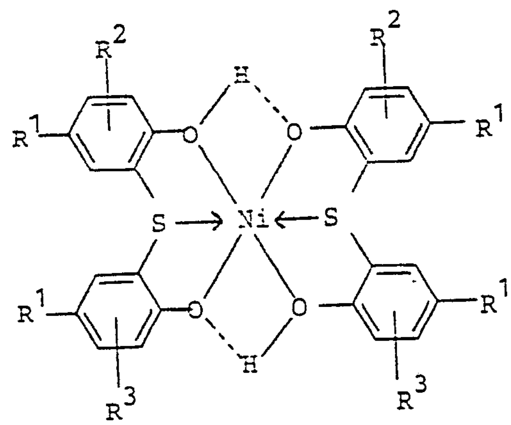

(V)

Die Herstellung der wasserunlöslichen Nickelkomplexe gemäß der Erfindung kann auf verschiedene Weise erfolgen. Bevorzugt werden jene Verfahren, bei denen das Bisphenol sowie gegebenenfalls ein zusätzlicher Ligand in einem niedrigsiedenden, mit Wasser nicht mischbaren Lösungsmittel vorliegen und das Nickelsalz über eine wäßrige oder zumindest teilweise wäßrige Phase eingebracht wird. Die Umsetzung wird dabei im allgemeinen unter starken Rühren durchgeführt und so lange fortgesetzt, bis aus der wäßrigen Phase kein Nickel mehr aufgenommen wird. Zur Beschleunigung der Umsetzung kann das Gemisch erhitzt, bzw. ein Teil des Lösungsmittels abdestilliert werden. Nach beendeter Umsetzung wird die wäßrige, gegebenenfalls, überschüssiges Nickelsalz enthaltende Phase abgetrennt.

Die Umsetzung erfolgt bevorzugt bei pH-Werten oberhalb 7, bezogen auf die wäßrige Phase. Als Base kann ein Alkalihydroxid, ein Alkalialkoholat, ein Amin oder Amidin, ein Alkaliphenolat, ein wasserlösliches Carbonat oder ein wasserlösliches Salz einer Carbonsäure verwendet werden.

Als Fremdligand L, der gegebenenfalls in das Nickelchelat mit eingebaut wird, kommt beispielsweise in Frage : ein bevorzugt tertiäres Mono- oder Diamin oder Amidin, z. B. Ethyldiisopropylamin, Bis-2-hydroxyethylcyclohexylamin, Pyridin, Diazabicyclo [2,2,2] octan, 1,8-Diazabicyclo [5,4,0]-7-undecen, 1,5-Diazabicyclo [4,3,0] non-5-en, ein Di- oder Triester der phosphorigen Säure, z. B. Tributylphosphit, ein aliphatischer, cycloaliphatischer oder araliphatischer Alkohol, ein Glykolmonoether, bzw. das entsprechende Anion.

Für die Herstellung der Nickelchelate gemäß der Erfindung werden nachstehend einige Beispiele angegeben.

Nickelkomplex 1

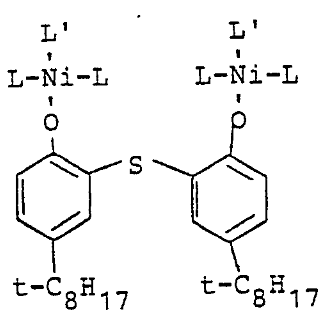

L = $HOCH_3$
L' = $OCH_3$

Die Herstellung erfolgt nach den Angaben der DE-OS 1 768 258 durch Kochen der Ausgangsverbindungen (Verbindung 1 + Nickelacetat im Molverhältnis 1:2) in Methanol in Gegenwart der berechneten Menge Na-methylat. Der Komplex ist blaßgrün gefärbt und in Toluol oder Ethylacetat glatt löslich.

Nickelkomplex 2

11,8 g (26 mMol) Verbindung 1 (hergestellt nach den Angaben in GB 858 890, Fp : 134-135 °C, 18,4 ml (108 mMol) Ethyldiisopropylamin, 80 ml Toluol und eine Lösung von 12,4 g (54 mMol) Nickelchlorid in 20 ml Wasser wurden nach Zugabe von 2,4 g NaOH in 10 ml Wasser 24 h am Dampfbad gerührt. Die organische Phase (Toluol) wurde von der Wasserphase abdekantiert und eingedampft. Ausbeute 11,1 g (80 % d. Theorie)

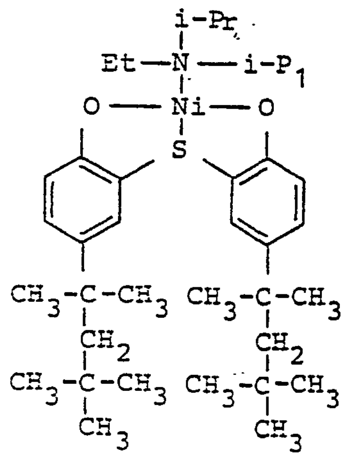

Et = $C_2H_5$

i-Pr = 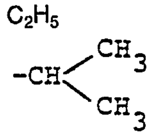

Nickelkomplex 3

Zu einer Suspension des in Beispiel 1 der DE-OS 2 042 652 beschriebenen [2,2'-Thiobis(4-t-octylphenolats)]-aquonickel (II)-chelates (hergestellt aus 11,8 g Verbindung 1 und 6,2 g Nickelchlorid) in 120 ml Toluol wurden 1,5 g Diazabicyclo [2,2,2] octan eingetragen. Nach 2 h Erhitzen unter Rückfluß wurde die gebildete klare Lösung unter vermindertem Druck eingedampft. Es wurden 12,7 g (95 % d. Theorie) blaßgrüne, in Ethylacetat vollständig lösliche Kristalle erhalten.

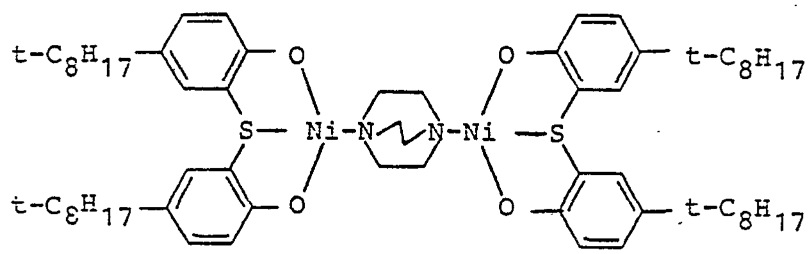

Nickelkomplex 4

Zu einer Suspension des in Beispiel 1 der DE-OS 2 042 652 beschriebenen [2,2'-Thiobis(4-t-octylphenolato)]-aquonickel(II)-chelates in 120 ml Toluol wurden unter Rühren am Rückfluß 11,8 g 2,2'-Thiobis(4-t-octylphenol) (= Verbindung 1) in 80 ml Toluol zugefügt. Nach 3 h Rückfluß war alles gelöst. Die Lösung wurde unter vermindertem Druck eingeengt. Der Rückstand wurde aus Toluol/Petrolether umgefällt. Ausbeute 22,2 g (90 % d. Theorie) blaßgrünes in Ethylacetat glatt lösliches Pulver.

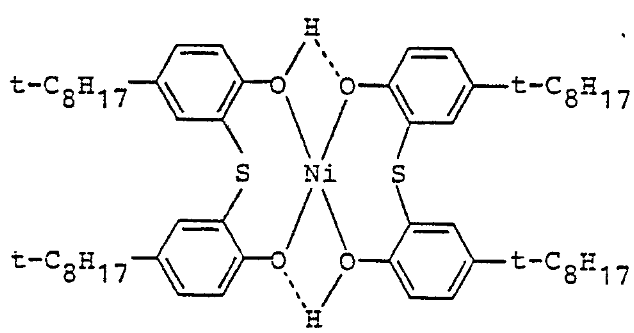

Nickelkomplex 5

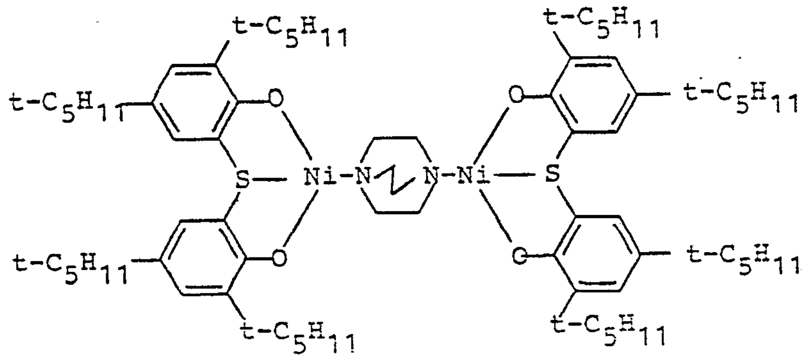

Herstellung analog Nickelkomplex 3 aus Verbindung 3, Nickelchlorid und Diazabicyclo[2,2,2]octan ; in Ethylacetat klar lösliche hellgrüne Kristalle.

Nickelkomplex 6

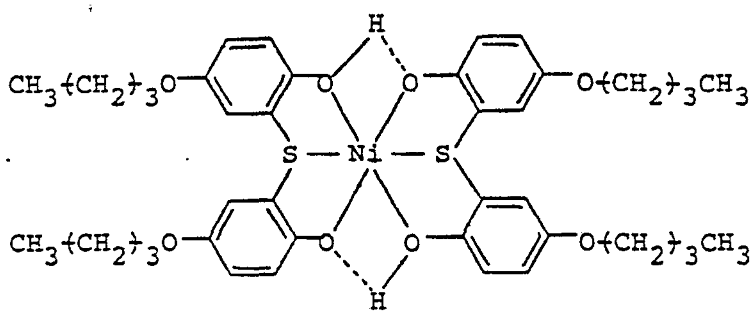

Herstellung analog Nickelkomplex 4 aus Verbindung 6 ; in Dichlormethan klar lösliches gelblichgrünes Kristallpulver.

Nickelkomplex 7

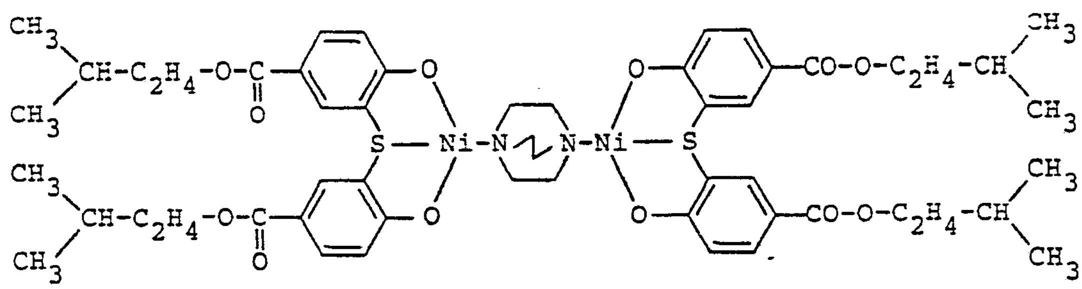

Herstellung analog Nickelkomplex 3 aus Verbindung 11, Nickelchlorid und Diazabicyclo[2,2,2]octan ; grünes in Ethylacetat lösliches Pulver.

Die Verwendung hydrophober Nickelkomplexe von 2,2'-Thiobisphenolen zur Stabilisierung von Polyolefinen oder Kautschuk sowie zur Verbesserung von deren Anfärbbarkeit ist bekannt [DE-OS 2 009 112, DE-OS 2 017 044, DE-OS 2 302 230].

Die Einlagerung von Nickelkomplexen aus 2,2'-Thiobisphenolen in fotografische Schichten, z. B. in Bildempfangsschichten für die Verwendung im Farbdiffusionsübertragungsverfahren ist jedoch nicht bekannt und führt zu überraschenden Ergebnissen.

Bildempfangsschichten für die Verwendung im Farbdiffusionsübertragungsverfahren enthalten zur Festlegung der üblicherweise anionischen Bildfarbstoffe kationische niedermolekulare micellbildende oder hochmolekulare, entweder in gelöster Form oder in Latexform vorliegende kationische Beizmittel.

Es ist bekannt, daß anionische Bildfarbstoffe bei der Festlegung auf kationischen Beizmitteln eine beträchtliche Einbuße an Lichtechtheit erleiden. Der Lichtechtheitsverlust ist stark von der Struktur und der Einlagerungsform des kationischen Beizmittels abhängig und hängt in schwer vorhersehbarer Weise mit der Konstitution des festgelegten Bildfarbstoffes zusammen.

Zur Verbesserung der Lichtechtheit von auf kationischen Beizmitteln festgelegten Bildfarbstoffen

wurde in DE-OS 2 740 719 deren nachträgliche Metallisierung mit Übergangsmetallionen vorgeschlagen.

Diese Maßnahme führt zwar in vielen Fällen zu einer beträchtlichen Verbesserung der Lichtechtheit, ist jedoch nicht generell erfolgreich und führt zudem in vielen Fällen zu nicht vorhersehbaren Nachteilen, z. B. Verfärbung der Schicht bei Belichtung.

Des weiteren ist festzustellen, daß die Geschwindigkeit der Komplexierung des Bildfarbstoffes stark von der Art der Einlagerung der Metallionen in die Bildempfangsschicht abhängt. Die Metallionen sind in der Bildempfangsschicht vielfach in komplexer Form an eingelagerte niedermolekulare oder hochmolekulare sogenannte Metallspender gebunden (z. B. DE-OS 30 02 287) und mit zunehmender Beständigkeit des in der Bildempfangsschicht vorliegenden Metallspenderkomplexes nimmt die Geschwindigkeit der Komplexierung des Bildfarbstoffes drastisch ab, sodaß der Farbumschlag zum endgültigen Bildton, der mit der Komplexierung der Bildfarbstoffes einhergeht, innerhalb der vorgesehenen Bildentstehungszeit nicht abgeschlossen ist.

Die Ursache für die mit der Erfindung erzielten Verbesserungen ist im einzelnen nicht bekannt ; die Tatsache, daß die beobachtbaren Lichtechtheitsverbesserungen auch mit nicht metallisierten Bildfarbstoffen erzielt werden, ist am ehesten mit einer Singlett-Sauerstoff-abfangenden Wirkung der erfindungsgemäßen Metallisierungsmittel, bzw. der daraus nach Abgabe des Metallions verbleibenden Bisphenole zu erklären. Darüber hinaus kann angenommen werden, daß die Nickelkomplexe eine allgemein triplettdesaktivierende Wirkung besitzen.

In diesem Zusammenhang ist auf folgende Literaturstellen zu verweisen :

J. Griffiths, C. Hawkins, J. Chem. Soc. Perkin Trans. II 1977, Seite 747-752 ; J. Chem. Soc. Chem. Commun. 1972, S. 463-463 ; H. Kautsky, H. de Bruin, Natur-Wiss. 19 (1931) 1043, H. Kautsky et al., Ber. dt. chem. Ges. 66, 1588 (1933) ; P. B. Merkel, W. F. Smith, J. Phys. Chem. 84, 2834-40 (1979).

Die Verwendung von Nickelchelaten zur Stabilisierung von fotografischen Farbbildern ist mehrfach beschrieben worden. So ist in der DE-OS 2 853 826 die Verwendung von Nickelchelaten aus Thioamiden der Picolinsäure- oder Chinaldinsäurereihe zur Stabilisierung von Farbstoffen gegen Licht beschrieben. Die beträchtliche Eigenfärbung der dort beschriebenen Nickelchelate würde schon bei Mengenanteilen von 5 mMol/m$^2$ Bildempfangsschicht zu einer prohibitiven Anfärbung der Bildweißen führen.

Zur Verwendung in Bildempfangsschichten für das Farbdiffusionsübertragungsverfahren sind nur solche Nickelkomplexe geeignet, die noch in Mengenanteilen von 50 mMol/m$^2$ nicht zu einer Anfärbung der Bildempfangsschicht über Dichten von 0,07 gemessen hinter Blau-, Grün- oder Rotfilter führen. Sowohl die Nickelchelate der DE-OS 2 853 826 als auch die Ni-chelate, die in den deutschen Offenlegungsschriften 2 854 040, 2 928 042, 2 456 075, 2 846 938, 2 846 937, 2 853 866 beschrieben sind, erfüllen diese Ansprüche nicht.

Die beschriebenen Nickelkomplexe können in der verschiedenartigsten Weise verwendet werden, um Nickelionen diffusionsfest in Schichten einzulagern, ohne daß ihre Reaktivität gegenüber komplexbildenden Substanzen verloren geht. So können die Nickelkomplexe mit Bindemitteln wie beispielsweise Gelatine, Polyvinylalkohol, Cellulosederivaten, Polyacrylamiden vermischt werden und in Form dieser Mischungen zur Herstellung transparenter Schichten verwendet werden.

Die beschriebenen Nickelkomplexe eignen sich ferner als Metallspender für die Einlagerung in fotografische Schichten, in denen durch Reaktion der komplex gebundenen Nickelionen mit anderen organischen farblosen oder farbigen farbbildenden Komplexbildnern eine gleichmäßige oder bildmäßige Anfärbung erzeugt werden soll. Die Erzeugung einer gleichmäßigen Anfärbung dient beispielsweise dazu um nach der bildmäßigen Belichtung ein bei der Entwicklung eines integralen Aufzeichnungsmaterials entstehendes unerwünschtes Bild unsichtbar zu machen, z. B. ein in dem lichtempfindlichen Element zurückgehaltenes negatives Farbbild, wenn in der Bildempfangsschicht ein positives Farbbild erzeugt wird. Mit Vorteil werden die beschriebenen Nickelkomplexe aber in einer Kombination mit einer Bildempfangsschicht verwendet, die zusammen mit einem Schichtträger ein sogenanntes Bildempfangselement darstellt. Ein solches Bildempfangselement kann ein integraler Bestandteil eines mehrschichtigen lichtempfindlichen farbfotografischen Aufzeichnungsmaterials sein, in welchem Fall es nach der Entwicklung entweder mit dem ursprünglich lichtempfindlichen Element verbunden bleibt oder von jenem abgetrennt wird. Das Bildempfangselement kann aber auch zunächst als separates nicht lichtempfindliches fotografisches Material vorliegen, das erst während der Verarbeitung des lichtempfindlichen Aufzeichnungsmaterials mit jenem in Kontakt gebracht und gegebenenfalls anschließend wieder abgetrennt wird.

Bei den anfärbbaren Schichten gemäß der vorliegenden Erfindung handelt es sich um hydrophile Schichten, die einen wasserunlöslichen vorzugsweise elektroneutralen Nickelkomplex eines Bisphenols einer der Formeln I und II in Form einer feinverteilten, gegebenenfalls ölbildnerhaltigen Dispersion enthalten. Die anfärbbaren Schichten können weiterhin Beizmittel für diffundierende anionische Farbstoffe enthalten oder mit entsprechenden Beizmittelschichten in unmittelbaren Kontakt stehen.

Als Beizmittel für die anionischen Bildfarbstoffe können grundsätzlich alle bekannten kationischen niedermolekularen oder hochmolekularen Beizen verwendet werden, z. B. mindestens einen langkettigen Alkyl- oder Aralkylrest enthaltende quartäre Ammonium- oder Phosphoniumsalze, wasserlösliche quarternierte Polyurethane oder Polyharnstoffe, schließlich Quartärsalzgruppen enthaltende vernetzte Latices. In diesem Zusammenhang ist z. B. auf die in DE-OS 2 631 521 genannten kationischen Glycidylgruppen enthaltenden Polyurethane zu verweisen.

Durch Kombination eines kationischen Polyurethans der zuletzt erwähnten Art (DE-OS 2 631 521) mit einem hydrophoben Nickelchelat gemäß der vorliegenden Erfindung lassen sich anfärbbare Schichten mit erheblichen Vorteilen herstellen, insbesondere im Hinblick auf das hohe Lichtechtheitsniveau und den klaren Farbton der Farbüberträge, wenn die anfärbbare Schicht als Bildempfangsschicht bei der Herstellung von Farbübertragungsbildern verwendet wird.

Zur Herstellung der anfärbbaren Schichten gemäß der Erfindung werden die hydrophoben Nickelchelate in dispergierter Form in Gegenwart eines hydrophilen Bindemittels, bevorzugt Gelatine, in eine Schicht eingelagert. Die Dispersionen werden hierzu auf an sich bekannte Weise hergestellt und enthalten bevorzugt einen neutralen Ölbildner, z. B. N,N-Diethyllaurinsäureamid, Trikresylphosphat, Dibutylphthalat oder Ester mehrwertiger Alkohole mit langkettigen Fettsäuren.

Der Gehalt an Ölbildner kann 10-200 Gew.-% bezogen auf Nickelchelat, betragen.

Die Bildempfangselemente können in üblicher Weise gehärtet werden, um ihre Kratzfestigkeit zu verbessern, sowie um die Flüssigkeitsaufnahme während der Verarbeitung mit einem alkalischen Medium zu begrenzen.

Die Stabilität der dispergiert eingelagerten Nickelchelate ist groß genug um zu verhindern, daß während einer längeren Lagerung freie Nickelionen in die hydrophile Bindemittelphase austreten können und dort gegebenenfalls bis zum eingelagerten Farbabspalter diffundieren können, womit ein Verlust an Diffusionsfähigkeit sowie an fotografischer Empfindlichkeit verbunden wäre.

Die hydrophoben Nickelchelate sind daher besonders geeignet zur Herstellung von integralen Monoblatt- oder Einzelblattmaterialien, bei denen das Bildempfangselement zusammen mit den Schichten des lichtempfindlichen Elementes in einem geschlossenen Schichtverband vorliegt und von jenen gegebenenfalls ausschließlich durch eine der optischen Trennung dienende opazifizierende Schicht getrennt ist.

Das erfindungsgemäße fotografische Material mit der die beschriebenen Nickelkomplexe enthaltenden anfärbbaren Schicht ist als Bildempfangsmaterial für jede Art von fotografischen Farbdiffusionsübertragungsverfahren geeignet, bei denen diffundierende Bildfarbstoffe oder auch diffundierende Farbbildner (Bildfarbstoffvorläufer) mit zur Chelatbildung befähigten Substituenten verwendet bzw. bildmäßig freigesetzt und auf eine Bildempfangsschicht übertragen werden können. Nach dem erfolgten Übertrag weist demnach ein solches fotografisches Material in der Bildempfangsschicht eine bildgemäße Verteilung eines oder mehrerer derartiger Farbstoffe auf.

Gemäß einer vorteilhaften Ausgestaltung der Erfindung kann das fotografische Material außer dem Bildempfangselement mit den beschriebenen hydrophoben Nickelkomplexen ein schichtförmiges lichtempfindliches Element aufweisen, das mindestens eine Schicht mit einem vorzugsweise sauren Farbstoff oder einer entsprechenden Vorläuferverbindung sowie mindestens eine lichtempfindliche Schicht, insbesondere eine lichtempfindliche Silberhalogenidemulsionsschicht enthält. Die erwähnten Farbstoffe bzw. Farbstoffvorläuferverbindungen werden nachfolgend zusammenfassend als farbgebende Verbindungen bezeichnet. In vorteilhafter Weise kann das fotografische Material mit der Bildempfangsschicht aus den erfindungsgemäßen Nickelkomplexen auch mehrere lichtempfindliche Silberhalogenidemulsionsschichten unterschiedlicher Spektralempfindlichkeit enthalten sowie weitere nicht lichtempfindliche Schichten wie Zwischenschichten, Deckschichten und andere Schichten der verschiedensten Funktionen, wie sie bei mehrschichtigen farbfotografischen Aufzeichnungsmaterialien üblich sind.

Fotografische Materialien, die eine Bildempfangsschicht mit erfindungsgemäßen Nickelkomplexen aufweisen, d. h. Bildempfangsmaterialien und insbesondere farbfotografische Aufzeichnungsmaterialien, die als integralen Bestandteil ein solches Bildempfangsmaterial enthalten, können darüber hinaus noch saure Schichten und sogenannte Brems- oder Verzögerungsschichten enthalten, die zusammen ein sogenanntes, kombiniertes Neutralisationssystem bilden. Ein solches Neutralisationssystem kann in bekannter Weise zwischen dem Schichtträger und der darauf angeordneten Bildempfangsschicht angeordnet sein oder an einer anderen Stelle im Schichtverband, z. B. oberhalb der lichtempfindlichen Schichten, d. h. jenseits dieser lichtempfindlichen Schichten von der Bildempfangsschicht aus betrachtet. Das Neutralisationssystem ist normalerweise so orientiert, daß sich die Brems- oder Verzögerungsschicht zwischen der Säureschicht und der Stelle befindet, an der die alkalische Entwicklungsflüssigkeit oder -paste zur Einwirkung gebracht wird. Solche Säureschichten, Bremsschichten bzw. aus beiden bestehende Neutralisationsschichten sind beispielsweise bekannt aus US 2 584 030, US 2 983 606, US 3 362 819, US 3 362 821, DE-OS 2 455 762, DE-OS 2 601 653, DE-OS 2 601 653, DE-OS 2 652 464, DE-OS 2 716 505, DE-OS 2 816 878. Ein solches Neutralisationssystem kann in bekannter Weise auch zwei oder mehrere Bremsschichten enthalten.

Das fotografische Material kann in einer besonderen Ausgestaltung des weiteren eine oder mehrere für wäßrige Flüssigkeiten durchlässige pigmenthaltige opake Schichten enthalten. Diese können zwei Funktionen erfüllen. Einerseits kann hierdurch der unerwünschte Zutritt von Licht zu lichtempfindlichen Schichten unterbunden werden und andererseits kann eine solche Pigmentschicht insbesondere, wenn ein helles oder weißes Pigment, z. B. $TiO_2$, verwendet wurden, für das erzeugte Farbbild einen ästhetisch angenehmen Hintergrund bilden. Integrale farbfotografische Aufzeichnungsmaterialien mit einer solchen Pigmentschicht sind bekannt, z. B. aus US 2 543 181 und DE-AS 1 924 430.

Anstelle einer vorgebildeten opaken Schicht können auch Mittel vorgesehen sein, um eine solche Schicht erst im Verlauf des Entwicklungsverfahrens zu erzeugen. Entsprechend den beiden erwähnten

Funktionen können derartige Pigmentschichten aus zwei oder mehreren Teilschichten aufgebaut sein, von denen eine beispielsweise ein weißes Pigment und die andere beispielsweise ein dunkles, Licht absorbierendes Pigment, z. B. Ruß, enthält.

Bei einer besonders bevorzugten Ausgestaltung der Erfindung ist das fotografische Material ein integrales farbfotografisches Aufzeichnungsmaterial für die Durchführung des Farbdiffusionsübertragungsverfahrens und weist beispielsweise folgende Schichtelemente auf :

1) einen transparenten Schichtträger ;
2) eine Bildempfangsschicht ;
3) eine lichtundurchlässige Schicht (Pigmentschicht) ;
4) ein lichtempfindliches Element mit mindestens einer lichtempfindlichen Silberhalogenidemulsionsschicht und mindestens einer dieser zugeordneten farbgebenden Verbindung ;
5) eine Bremsschicht ;
6) eine saure Polymerschicht ;
7) einen transparenten Schichtträger.

Dieses Material kann dabei in der Weise zusammengesetzt werden, daß getrennt voneinander zwei verschiedene Teile hergestellt werden, nämlich der lichtempfindliche Teil (Schichtelemente 1 bis 4) und das Abdeckblatt (Schichtelemente 5 bis 7), die dann schichtseitig aufeinander gelegt und miteinander verbunden werden, gegebenenfalls unter Verwendung von Abstandsstreifen, so daß zwischen den beiden Teilen ein Raum für die Aufnahme einer genau bemessenen Menge einer Entwicklungsflüssigkeit gebildet wird. Die Schichtelemente 5 und 6, die zusammen das Neutralisationssystem bilden, können auch, allerdings in vertauschter Reihenfolge, zwischen dem Schichtträger und der Bildempfangsschicht des lichtempfindlichen Teiles angeordnet sein.

Es können Mittel vorgesehen sein, um eine Entwicklungsflüssigkeit zwischen zwei benachbarte Schichten des integralen Aufzeichnungsmaterials einzuführen, z. B. in Form eines seitlich angeordneten, aufspaltbaren Behälters, der bei Einwirkung mechanischer Kräfte seinen Inhalt zwischen zwei benachbarte Schichten des Aufzeichnungsmaterials, im vorliegenden Fall zwischen den lichtempfindlichen Teil und das Abdeckblatt, ergießt.

Das lichtempfindliche Element kann im Falle eines integralen Aufzeichnungsmaterials ebenfalls wesentlicher Bestandteil des erfindungsgemäßen fotografischen Materials sein. Falls das erfindungsgemäße fotografische Material selbst nicht lichtempfindlich ist, sondern im wesentlichen nur aus Schichtträger und Bildempfangsschicht besteht, wird es während des Entwicklungsverfahrens in Kontakt gebracht mit dem lichtempfindlichen Element eines lichtempfindlichen Aufzeichnungsmaterials. Das lichtempfindliche Element enthält im Falle eines Einfarbstoffübertragungsverfahrens eine lichtempfindliche Silberhalogenidemulsionsschicht und dieser zugeordnet eine farbgebende Verbindung. Dabei kann sich die farbgebende Verbindung in einer zu der Silberhalogenidemulsionsschicht benachbarten Schicht oder in der Silberhalogenidemulsionsschicht selbst befinden, wobei im letzteren Fall die Farbe des Bildfarbstoffes bevorzugt so ausgewählt wird, daß der überwiegende Absorptionsbereich der farbgebenden Verbindung nicht übereinstimmt mit dem überwiegenden Empfindlichkeitsbereich der Silberhalogenidemulsionsschicht. Zur Herstellung mehrfarbiger Übertragsbilder in naturgetreuen Farben enthält das lichtempfindliche Element jedoch drei derartige Zuordnungen von farbgebender Verbindung und lichtempfindlicher Silberhalogenidemulsionsschicht, wobei in der Regel der Absorptionsbereich des aus der farbgebenden Verbindung resultierenden Bildfarbstoffes mit dem Bereich der spektralen Empfindlichkeit der zugeordneten Silberhalogenidemulsionsschicht im wesentlichen übereinstimmen wird. Günstig für die Erzielung einer möglichst hohen Empfindlichkeit ist es dann jedoch, wenn jeweils die farbgebende Verbindung in einer separaten Bindemittelschicht (gesehen in Richtung des bei der Belichtung einfallenden Lichtes) hinter der Silberhalogenidemulsionsschicht angeordnet ist oder eine Absorption aufweist, die von derjenigen des Bildfarbstoffes verschieden ist (« verschobene Bildfarbstoffe » — US 3 854 945). Eine Verschiebung der Absorption ergibt sich in der Regel in der Bildempfangsschicht durch Komplexbildung mit den darin vorliegenden Nickelionen, wenn Farbstoffe mit zur Chelatbildung befähigten Substituenten verwendet werden. Farbgebende Verbindungen mit solchen Farbstoffresten sind beispielsweise beschrieben in US 3 081 167 und DE-OS 2 740 719.

Bei den farbgebenden Verbindungen kann es sich um farbige Verbindungen handeln, die selbst diffusionsfähig sind und die bei der Behandlung der Schichten mit einer alkalischen Arbeitsflüssigkeit anfangen, zu diffundieren und lediglich an den belichteten Stellen durch die Entwicklung festgelegt werden. Die farbgebenden Verbindungen können aber auch diffusionsfest sein und im Verlauf der Entwicklung einen diffundierenden Farbstoff in Freiheit setzen.

Farbgebende Verbindungen, die a priori diffusionsfähig sind, sind beispielsweise bekannt aus den deutschen Patentschriften 1 036 640, 1 111 936 und 1 196 075. Die dort beschriebenen sogenannten Farbstoffentwickler enthalten im gleichen Molekül einen Farbstoffrest und eine Gruppierung, die in der Lage ist, belichtetes Silberhalogenid zu entwickeln.

Unter den bisher bekannt gewordenen Verfahren zur Herstellung farbfotografischer Bilder nach dem Farbdiffusionsübertragungsverfahren gewinnen in jüngster Zeit zunehmend solche an Bedeutung, die auf der Verwendung diffusionsfest eingelagerter, farbgebender Verbindungen beruhen, aus denen bei der Entwicklung diffundierende Farbstoffe oder Farbstoffvorläuferprodukte bildmäßig abgespalten und auf eine Bildempfangsschicht übertragen werden. Derartige nicht diffundierende farbgebende Verbindungen (Farbabspalter) sind beispielsweise in den folgenden Druckschriften beschrieben :

US 3 227 550, US 3 443 939, US 3 443 940, DE-OS 1 930 215, DE-OS 2 242 762, DE-OS 2 402 900, DE-OS 2 406 664, DE-OS 2 505 248, DE-OS 2 543 902, DE-OS 2 613 005, DE-OS 2 645 656, DE-OS 2 809 716, BE 861 241.

In den genannten Druckschriften werden sowohl solche Farbabspalter beschrieben, die bei Verwendung üblicher negativer Silberhalogenidemulsionen negative Farbbilder erzeugen, als auch solche, die bei Verwendung negativer Silberhalogenidemulsionen positive Farbbilder erzeugen. Im ersteren Fall bedarf es, falls positive Farbbilder erwünscht sind, entweder der Verwendung direkt positiver Silberhalogenidemulsionen oder, bei Verwendung von negativen Emulsionen, der Anwendung eines der bekannten Umkehrverfahren, z. B. nach dem Silbersalzdiffusionsverfahren (US 2 763 800) oder durch Verwendung von Verbindungen, die als Folge der Entwicklung Entwicklungsinhibitoren in Freiheit setzen.

Bei Verwendung der erfindungsgemäßen Bildempfangselemente werden bevorzugt Farbabspalter verwendet, die im Farbstoffrest zur Komplexbildung befähigte Substituenten enthalten. Hierunter sind besonders zu erwähnen Azofarbstoffe, die benachbart zur Azobindung chelatbildende Substituenten wie —OH, —NHR oder auch Ringstickstoffatome aufweisen. Farbgebende Verbindungen mit solchen Farbstoffresten sind beispielsweise beschrieben in den Research Disclosure Publikationen Nr. 17334 (Sept. 1978) und Nr. 18022 (April 1979), sowie in US 3 081 167, DE-OS 2 740 719 und in DE-OS 31 07 540, DE-OS 31 15 648 und DE-OS 31 17 243. Jedoch wird ebenso eine stabilisierende Wirkung erzielt, wenn Farbabspalter verwendet werden, die nicht nachkomplexierbare Farbstoffe liefern.

Beispiel 1

Ein Bildempfangsblatt gemäß Erfindung wurde hergestellt, in dem auf einen polyethylenkaschierten Papierträger nacheinander folgende Schichten aufgetragen wurden (Mengenangaben pro $m^2$) :

Schicht 1 :

4 g Gelatine
2 g Nickelkomplex 1
2,5 g Trikresylphosphat
0,03 g Saponin

Schicht 2 :

4 g kationisches Polyurethan gemäß Beispiel 3 der DE-OS 2 631 521
5 g Gelatine
0,02 g Saponin

Schicht 3 :

0,1 g Gelatine
0,2 g Soforthärtungsmittel der Formel

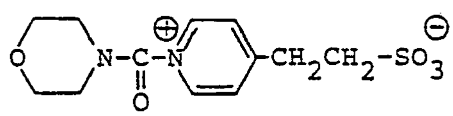

Beispiel 2

Es wurde wie in Beispiel 1 verfahren, jedoch mit dem Unterschied, daß Schicht 1 folgende Zusammensetzung hatte :

Schicht 1 :

4 g Gelatine
2,8 g Nickelkomplex 2
2,8 g Trikresylphosphat und
0,03 g Saponin

Beispiel 3

Es wurde wie in Beispiel 1 verfahren, jedoch mit dem Unterschied, daß Schicht 1 folgende Zusammensetzung hatte :

Schicht 1 :

4 g Gelatine
2,8 g Nickelkomplex 3
2,5 g Trikresylphosphat und
0,03 g Saponin

Beispiel 4

Es wurde wie in Beispiel 1 verfahren, jedoch mit dem Unterschied, daß Schicht 1 folgende Zusammensetzung hatte :

Schicht 1 :

4 g Gelatine
4,0 g Nickelkomplex 4
3,0 g Trikresylphosphat und
0,03 g Saponin

Beispiel 5

Es wurde wie in Beispiel 1 verfahren, jedoch mit dem Unterschied, daß Schicht 1 folgende Zusammensetzung hatte :

Schicht 1 :

4 g Gelatine
3,2 g Nickelkomplex 6
3,2 g Trikresylphosphat und
0,3 g Saponin.

Beispiel 6

Auf einen polyethylenkaschierten Papierträger wurden nacheinander folgende Schichten aufgetragen (Mengenangaben pro $m^2$) :

Schicht 1 :

5,5 g Gelatine
3,0 g kationisches Polyurethan gemäß Beispiel 3 der DE-OS 2 631 521
2,0 g Nickelkomplex 1
2,5 g Trikresylphosphat
0,03 g Saponin

Schicht 2 :

0,1 g Gelatine
0,2 g Soforthärtungsmittel der Formel

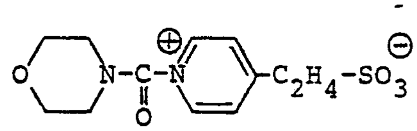

Beispiel 7

Ein Bildempfangsblatt gemäß dem Stand der Technik wurde hergestellt, indem wie in Beispiel 6 verfahren wurde, jedoch mit dem Unterschied, daß Schicht 1 folgende Zusammensetzung hatte :

Schicht 1 :

5,5 g Gelatine
3,0 g kationisches Polyurethan nach Beispiel 3 der DE-OS 2 631 521
0,3 g Saponin

Anwendungsbeispiel 1

Von folgenden nachkomplexierbaren Bildfarbstoffen wurden 0,03 molare Lösungen hergestellt und mit 1 % NaOH alkalisch gestellt.

Farbstoff A

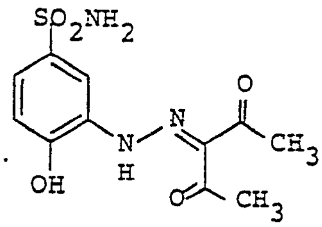

Farbstoff B

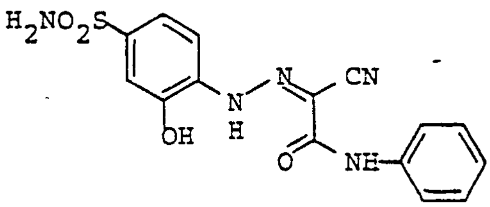

Farbstoff C

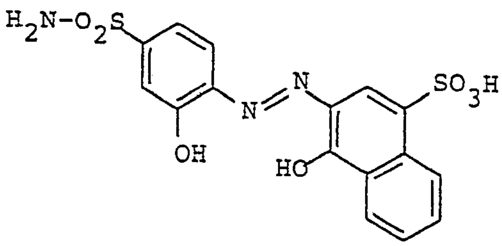

Farbstoff D

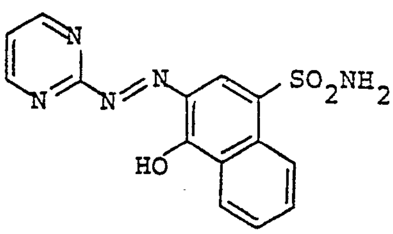

Farbstoff E

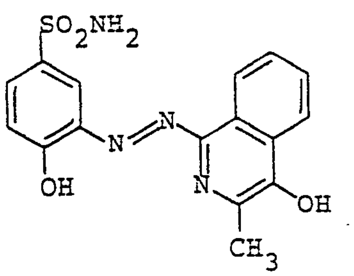

Farbstoff F (Farbstoff der DE-OS 31 07 540)

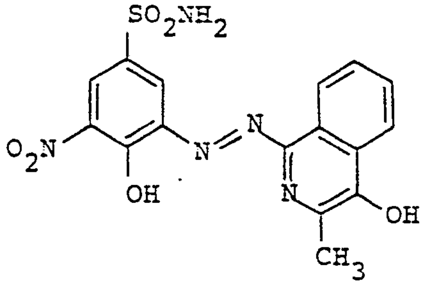

Farbstoff G (Farbstoff 3 der DE-OS 31 15 648)

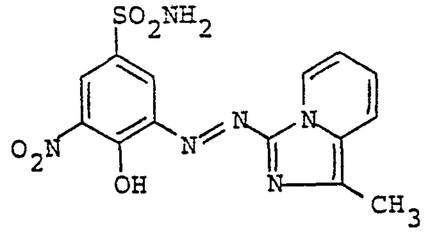

In diese Lösungen wurde je 1 Streifen von jedem der Bildempfangsblätter 1-6 eingetaucht, bis eine Dichte von 1,5, gemessen hinter komplementärfarbigem Filter, erreicht war. Bei Bildempfangsblatt 7 wurden die entsprechenden Streifen so lange eingetaucht, bis eine 30 s dauernde Nachbehandlung mit 2 %iger Nickelacetatlösung eine Dichte von 1,5 ergab.

Anschließend wurden die Streifen mit einer auf pH = 6 gestellten 2 %igen Natriumsuccinatlösung 1 min nachbehandelt und getrocknet.

Die Auswertung zeigt, daß die erwünschten Farbtöne im wesentlichen ohne Verzögerung gegenüber dem Stand der Technik erreicht werden und daß die Nuancen in allen Fällen denen entsprechen, die mit Bildempfangsblatt 7 bei Nachbehandlung mit Ni-acetat erhalten werden. Es treten keinerlei bräunliche Verfärbungen auf. Die Komplexierung erfolgt augenblicklich, ein allmählicher Farbumschlag ist nicht zu erkennen.

Zur Lichtechtheitsprüfung wurde je 1 Streifen mit Farbüberträgen der Farbstoffe A bis G auf den Bildempfangsblättern 1, 2 und 7 48 h einer Hochintensitätsbelichtung im Xenotest-Gerät unterworfen. Die nach Einwirkung von $4{,}8 \cdot 10^6$ lxh festgestellten prozentalen Dichteverluste, bezogen auf die Ausgangsdichte 1,5, sind in der folgenden Tabelle 1 zusammengefaßt:

Tabelle 1

| Bildempfangs- | Dichteverlust [%] | | | | | | |
|---|---|---|---|---|---|---|---|
| blatt | A | B | C | D | E | F | G |
| 1 | 20 | 10 | 5 | 10 | 28 | 30 | 20 |
| 2 | 48 | 11 | 4 | 10 | 38 | 30 | 35 |
| 7 | 50 | 22 | 7 | 25 | 40 | 45 | 30 |

Die Ergebnisse lassen den Schluß zu, daß die Lichtechtheit von Farbüberträgen aus nachkomplexierbaren anionischen Bildfarbstoffen durch Metallisierung mit hydrophoben Nickelchelaten gemäß der Erfindung verbessert werden kann.

Anwendungsbeispiel 2

Es wurde verfahren wie in Anwendungsbeispiel 1, jedoch unter Verwendung folgender nicht nachkomplexierbarer Bildfarstoffe:

Farbstoff H:

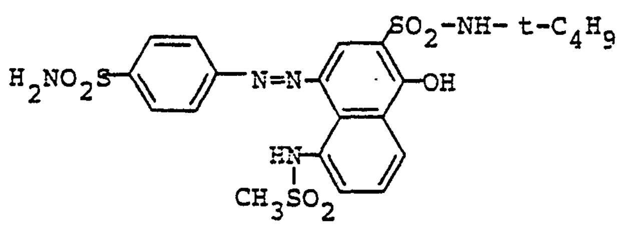

Farbstoff I:

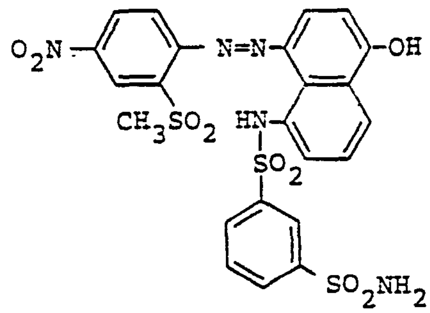

Farbstoff K:

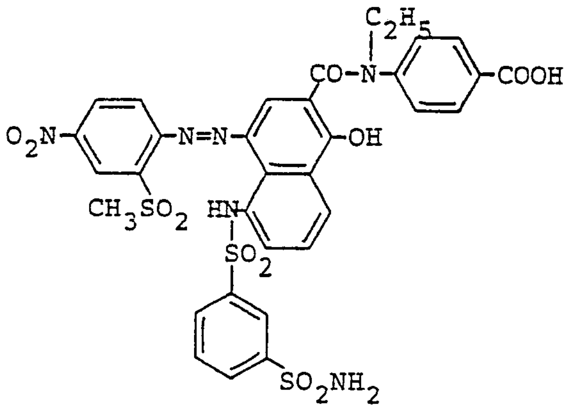

Zur Lichtechtheitsprüfung wurde je 1 Streifen mit Farbüberträgen der Farbstoffe H bis K auf den Bildempfangsblättern 2, 3 und 7 24 h einer Hochintensitätsbelichtung im Xenotest-Gerät unterworfen. Die nach Einwirkung von $2{,}4 \cdot 10^6$ lxh festgestellten prozentalen Dichteverluste, bezogen auf die Ausgangsdichte 1,5, sind in der folgenden Tabelle 2 zusammengefaßt:

Tabelle 2

| Bildempfangsblatt | Dichteverlust /%/ | | |
| --- | --- | --- | --- |
| | H | I | K |
| 2 | 21 | 57 | 23 |
| 3 | 21 | 49 | 21 |
| 7 | 40 | 71 | 35 |

Die Ergebnisse lassen den Schluß zu, daß die Lichtechtheiten von Farbüberträgen aus nichtkomplexierbaren anionischen Bildfarbstoffen mit hydrophoben Nickelchelaten gemäß der Erfindung verbessert werden können.

**Patentansprüche**

1. Fotografisches Material mit einer auf einem Schichtträger angeordneten durch organische Farbstoffe anfärbbaren Schicht, die ein Metallisierungsmittel für die Bildung von organischen Farbstoff-Metall-Komplexen enthält, dadurch gekennzeichnet, daß die durch organische Farbstoffe anfärbbare Schicht als Metallisierungsmittel einen aus einem 2,2'-Bisphenol einer der folgenden allgemeinen Formeln I und II gebildeten wasserunlöslichen Nickelkomplex enthält:

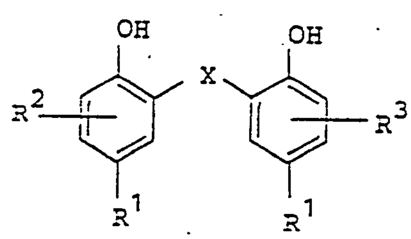

(I)

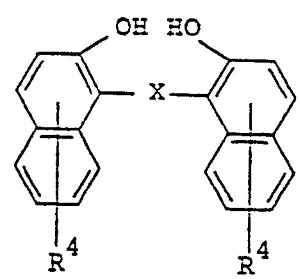

(II)

worin bedeuten

X 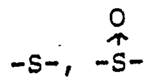 oder —$SO_2$— ;

$R^1$ einen Kohlenwasserstoffrest mit bis zu 18 C-Atomen, der entweder direkt oder über —O— angeknüpft ist,

Halogen oder eine Gruppe $R^4$,

$R^2$ Wasserstoff, Halogen, Alkyl oder Alkoxy mit bis zu 18 C-Atomen, Alkenyl, einen Rest zur Vervollständigung eines ankondensierten Benzolringes ; oder eine Gruppe $R^4$, falls nicht $R^1$ eine Gruppe $R^4$ bedeutet ;

$R^3$ einen Rest wie definiert für $R^2$ oder eine Gruppe der Formel

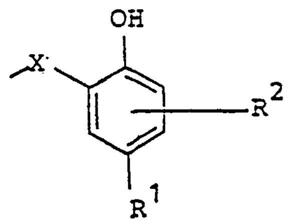

worin X, $R^1$ und $R^2$ die angegebene Bedeutung haben ;

$R^4$ 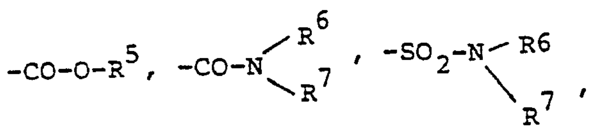

—NH—CO—$R^8$ oder —NH—$SO_2R^9$ ;

$R^5$ Alkyl, Aralkyl oder Cycloalkyl mit bis zu 8 C-Atomen ;

$R^6$, $R^7$ Wasserstoff, Alkyl, Aralkyl, Aryl, Cycloalkyl oder gemeinsam einen Rest zur Vervollständigung einer 5-, 6- oder 7-gliedrigen cyclischen Aminogruppe ;

$R^8$ Alkyl, Aralkyl, Aryl, —$OR^5$ oder

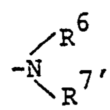

$R^9$ Alkyl, Aryl oder

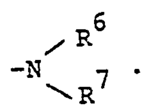

2. Fotografisches Material nach Anspruch 1, dadurch gekennzeichnet, daß die anfärbbare Schicht einen Nickelkomplex eines 2,2'-Bisphenols der Formel I enthält, worin $R^1$ Alkyl und $R^2$ und $R^3$ Wasserstoff, Chlor oder Alkyl bedeuten.

3. Fotografisches Material nach Anspruch 1, dadurch gekennzeichnet, daß die anfärbbare Schicht einen Nickelkomplex eines 2,2'-Bisphenols der Formel II enthält, worin $R^4$ eine Gruppe der Formel

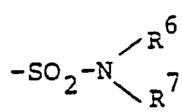

in 6-Stellung (des Naphthalinringes) bedeutet, wobei $R^6$ für Alkyl und $R^7$ für Wasserstoff oder Alkyl stehen.

4. Fotografisches Material nach einem der Ansprüche 1 bis 3, dadurch gekennzeichnet, daß die anfärbbare Schicht ein Beizmittel für diffundierende anionische Farbstoffe enthält oder mit einer entsprechenden Beizmittelschicht in unmittelbarem Kontakt steht.

5. Fotografisches Material nach einem der Ansprüche 1 bis 3, dadurch gekennzeichnet, daß die anfärbbare Schicht wesentlicher Teil eines Bildempfangselementes für das Farbdiffusionsverfahren ist.

6. Fotografisches Material nach Anspruch 5, dadurch gekennzeichnet, daß das die anfärbbare Schicht enthaltende Bildempfangselement integraler Bestandteil eines mehrschichtigen lichtempfindlichen farbfotografisches Aufzeichnungsmaterials ist.

**Claims**

1. Photographic material having a layer which can be dyed by organic dyes, contains a metallising agent for the formation of organic dye/metal complexes and is arranged on a layer support, characterised in that the layer which can be dyed by organic dyes contains, as the metallising agent, a water-insoluble nickel complex formed from a 2,2'-bisphenol of one of the following general formulae I and II :

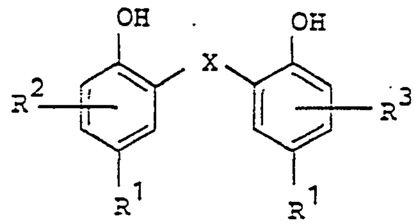

(I)

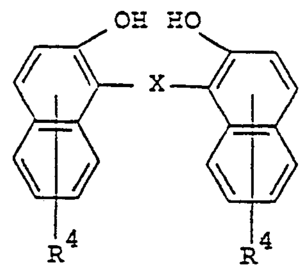

(II)

wherein

X denotes $-S-$, 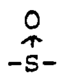 or $-SO_2-$;

$R^1$ denotes a hydrocarbon radical with up to 18 C atoms which is attached either directly or via —O—, halogen or a group $R^4$,

$R^2$ denotes hydrogen, halogen, alkyl or alkoxy with up to 18 C atoms, alkenyl, a radical for completing a fused-on benzene ring ; or a group $R^4$, unless $R^1$ denotes a group $R^4$ ;

$R^3$ denotes a radical as defined under $R^2$ or a group of the formula

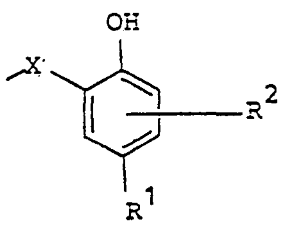

wherein

X, $R^1$ and $R^2$ have the abovementioned meaning ;

$R^4$ denotes

$-CO-O-R^5$, 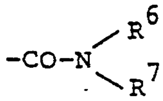, 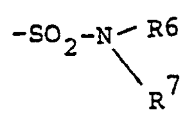,

$-NH-CO-R^8$ or $-NH-SO_2-R^9$ ;

$R^5$ denotes alkyl, aralkyl or cycloalkyl with up to 8 C atoms ;

$R^6$ and $R^7$ denote hydrogen, alkyl, aralkyl, aryl or cycloalkyl or together denote a radical for completing a 5-, 6- or 7-membered cyclic amino group ;

$R^8$ denotes alkyl, aralkyl, aryl, $-OR^5$ or

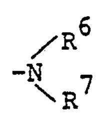,

and

$R^9$ denotes alkyl, aryl or

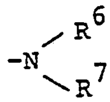 .

2. Photographic material according to Claim 1, characterised in that the dyeable layer contains a nickel complex of a 2,2'-bisphenol of the formula I, wherein $R^1$ denotes alkyl and $R^2$ and $R^3$ denote hydrogen, chlorine or alkyl.

3. Photographic material according to Claim 1, characterised in that the dyeable layer contains a nickel complex of a 2,2'-bisphenol of the formula II, wherein $R^4$ denotes a group of the formula

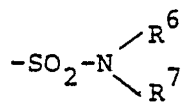

in the 6-position (of the naphthalene ring), wherein $R^6$ represents alkyl and $R^7$ represents hydrogen or alkyl.

4. Photographic material according to one of Claims 1 to 3, characterised in that the dyeable layer contains a mordant for diffusing anionic dyes or is in direct contact with an appropriate mordant layer.

5. Photographic material according to one of Claims 1 to 3, characterised in that the dyeable layer is an essential part of an image-receiving element for the dye diffusion process.

6. Photographic material according to Claim 5, characterised in that the image-receiving element containing the dyeable layer is an integral part of a multilayer light-sensitive colour-photographic recording material.

## Revendications

1. Matériel photographique avec une couche disposée sur un support de couche et pouvant être teinte par des colorants organiques, laquelle contient un agent de métallisation pour la formation de complexes colorant organique-métal, caractérisé en ce que la couche pouvant être teinte par des colorants organiques contient comme agent de métallisation un complexe de nickel insoluble dans l'eau formé à partir d'un 2,2'-bisphénol répondant à une des formules générales suivantes I et II :

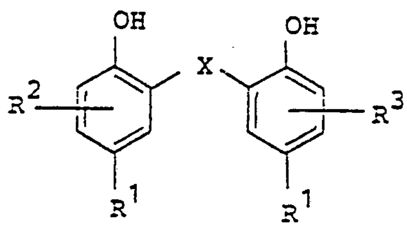

(I)

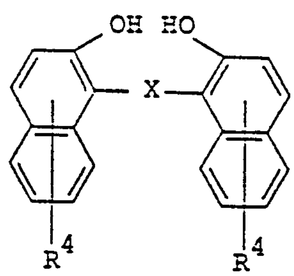

(II)

dans lesquelles

X signifie

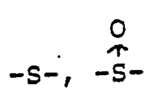 ou —$SO_2$—,

$R^1$ un radical hydrocarboné ayant jusqu'à 18 atomes de carbone, relié directement ou bien par —O—, de l'halogène ou un groupe $R^4$,

$R^2$ de l'hydrogène, halogène, alcoyle ou alcoxy ayant jusqu'à 18 atomes de carbone, alcényle, un radical pour compléter un noyau benzénique condensé, ou bien un groupe $R^4$ au cas où $R^1$ ne signifie pas un groupe $R^4$,

$R^3$ un radical tel que défini pour $R^2$ ou un groupe de formule :

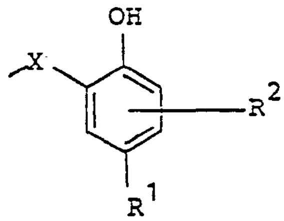

dans laquelle X, $R^1$ et $R^2$ ont la signification indiquée,

$-CO-O-R^5$, 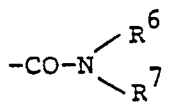 , 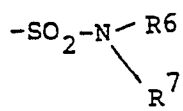 ,

$-NH-CO-R^8$ ou $-NH-SO_2R^9$,

$R^5$ un alcoyle, aralcoyle ou cycloalcoyle ayant jusqu'à 8 atomes de carbone,

$R^6$, $R^7$ de l'hydrogène, alcoyle, aralcoyle, aryle, cycloalcoyle ou ensemble un radical pour compléter un groupe amino cyclique à 5, 6 ou 7 chaînons,

$R^8$ un alcoyle, aralcoyle, aryle, $-OR^5$ ou

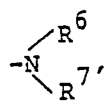,

$R^9$ un alcoyle, aryle ou

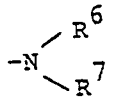.

2. Matériel de photographie selon la revendication 1, caractérisé en ce que la couche pouvant être teinte contient un complexe de nickel d'un 2,2'-bisphénol de formule I dans laquelle $R^1$ signifie un alcoyle et $R^2$ et $R^3$ de l'hydrogène, du chlore ou un alcoyle.

3. Matériel photographique selon la revendication 1, caractérisé en ce que la couche pouvant être teinte contient un complexe de nickel d'un 2,2'-bisphénol de formule II, dans laquelle $R^4$ représente un groupe de formule :

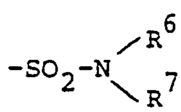

en position 6 (du noyau de naphtalène), $R^6$ représentant un alcoyle et $R^7$ de l'hydrogène ou un alcoyle.

4. Matériel photographique selon l'une des revendications 1 à 3, caractérisé en ce que la couche pouvant être teinte contient un mordant pour colorants anioniques diffusibles, ou bien est mise en contact direct avec une couche correspondante de mordant.

5. Matériel photographique selon l'une des revendications 1 à 3, caractérisé en ce que la couche pouvant être teinte est une partie essentielle d'un élément récepteur d'image pour le procédé de diffusion de la couleur.

6. Matériel photographique selon la revendication 5, caractérisé en ce que l'élément récepteur d'image contenant la couche pouvant être teinte est un constituant intégral d'un matériel d'enregistrement de photographie en couleur à couche multiple et sensible à la lumière.